Europäisches Patentamt

European Patent Office

Office européen des brevets

(11) Veröffentlichungsnummer: **0 261 402 B1**

(12) **EUROPÄISCHE PATENTSCHRIFT**

(45) Veröffentlichungstag der Patentschrift: **18.03.92**

(21) Anmeldenummer: **87112103.4**

(22) Anmeldetag: **20.08.87**

(51) Int. Cl.5: **A61L 15/16, A61M 37/00**

(54) **Transdermales therapeutisches System, seine Verwendung und Verfahren zu seiner Herstellung.**

(30) Priorität: **28.08.86 DE 3629304**

(43) Veröffentlichungstag der Anmeldung:
**30.03.88 Patentblatt 88/13**

(45) Bekanntmachung des Hinweises auf die
Patenterteilung:
**18.03.92 Patentblatt 92/12**

(84) Benannte Vertragsstaaten:
**AT BE CH DE ES FR GB GR IT LI LU NL SE**

(56) Entgegenhaltungen:
**EP-A- 0 117 027**
**EP-A- 0 170 010**
**GB-A- 1 361 289**
**US-A- 3 797 494**

(73) Patentinhaber: **LTS Lohmann Therapie-**
**Systeme GmbH & Co. KG**
**Irlicherstrasse 55**
**W-5450 Neuwied 12(DE)**

(72) Erfinder: **Hoffmann, Annegrete, geb. Ross-**
**bach**
**Burghofstrasse 123**
**W-5450 Neuwied 22(DE)**

(74) Vertreter: **Neidl-Stippler, Cornelia, Dr.**
**Rauchstrasse 2**
**W-8000 München 80(DE)**

**Beschreibung**

Die Erfindung betrifft ein therapeutisches System zur Verabreichung von Wirkstoffen an die Haut mit einer der Haut abgewandten Rückschicht; einem flüssigen Wirkstoff oder flüssige Wirkstoffzubereitung enthaltenden Wirkstoffdepot; einer die Wirkstoffabgabe steuernden Matrix und einer haftklebenden Fixierungseinrichtung für das therapeutische System auf der Haut, seine Verwendung sowie Verfahren zu seiner Herstellung.

Therapeutische Systeme zur transdermalen Verabreichung von Arzneimitteln geben einen oder mehrere Wirkstoffe in vorherbestimmter Rate kontinuierlich über einen festgelegten Zeitraum an einem festgelegten Anwendungsort an die Haut ab.

Diese Systeme sind therapeutische Präzisionsinstrumente, welche eine kontinuierliche Wirkstoff-Freisetzung sicherstellen.

Derartige therapeutische Systeme können sowohl topische als auch systemische Wirkung entfalten und die Vielfalt der auf diese Weise applizierbaren Wirkstoffe und ihre unterschiedlichen chemischen, physikalischen und pharmakologischen Eigenschaften stellen stets neue Anforderungen an die Herstellung derartiger Systeme.

Üblicherweise weisen diese transdermalen Systeme mindestens ein Wirkstoff-Reservoir, in dem die Wirkstoff-Substanz in fester, flüssiger oder molekulardisperser Form vorliegt und eine Adhäsionsschicht, durch die das System mit der Haut eng verbunden wird und durch welche der Wirkstoff-Transport erfolgt, eine Steuermembran und Schutzschichten/Deckschichten, die für den Wirkstoff im wesentlichen impermeabel sind, auf.

Die bekannten Systeme sind aufwendig in der Herstellung und kompliziert aufgebaut.

Ein Problem herkömmlicher Systeme besteht darin, leicht flüchtige Wirkstoffe zu verarbeiten, da die Verdampfung des Wirkstoffes schwer während der Herstellung zu kontrollieren ist.

Thermisch empfindliche Wirkstoffe sind bei thermisch zu behandelnden Matrices bzw. therapeutischen Systemen, die mit Wärmebehandlungsschritten hergestellt werden, nur beschränkt einsetzbar.

Es ist bereits versucht worden, reinen Wirkstoff in fein kristalliner Form in ein haftklebendes Polymerisat einzubringen, so daß der feinverteilte feinkristalline Wirkstoff sich als Depotkristalle in der klebenden Matrixschicht mit der Zeit lösen wird (DE-OS 35 00 508). Dieses Verfahren eignet sich schlecht für flüchtige und thermisch empfindliche Wirkstoffe, da es thermische Behandlungsschritte umfasst.

Ein anderer Versuch, die Kapazität derartiger therapeutischer Systeme zu erhöhen, besteht darin, Wirkstoffdepots in Form von Mikrokapseln, die von einer Steuermembran umschlossen sind, in eine Haftklebeschicht eines derartigen Systems einzubetten (siehe US-PS 3 598 123 und US-PS 3 731 683). Die Herstellung derartiger mit Steuermembranen umgebener Mikrokapseln ist außerordentlich aufwendig und teuer und läßt sich nicht für viele Wirkstoffe durchführen. Das Untermischen der wirkstoffhaltigen Mikrokapseln unter ein Reservoirmaterial stellt einen weiteren aufwendigen Verfahrensschritt dar, wobei die Mikrokapseln während dieses Schrittes leicht zerstört oder beschädigt werden können, was zu einer nicht zufriedenstellenden Konstanz des Wirkstoffgehalts im fertigen therapeutischen System führen kann. Das Verfahren der US-PS 3 598 123 ist für flüssige Wirkstoffe schwierig durchzuführen, insbesondere dann, wenn der flüssige Wirkstoff leicht flüchtig ist.

Aus der DE-PS 3 424 837 ist ein Depotpflaster bekannt geworden, das sich für flüssige Materialien einsetzen läßt und eine Deckfolie, einen flüssigen Wirkstoff in einem ausgewölbten Bereich der Deckfolie und eine den Wirkstoff abdeckende, für den Wirkstoff durchlässige Steuermembran aufweist. Dabei liegt zwischen der Deckfolie und der Steuermembran eine Wirkstoffverteilungs-Einrichtung, nämlich ein Vlies, das die Wirkstoff-Flüssigkeit gleichmäßig auf der Steuermembran verteilt und über einen größeren Flächenbereich wirksam werden läßt. Beim Depotpflaster der DE-PS 3 424 837 sind Deckfolie und Steuermembran jeweils an ihren Außenbereichen zusammengeschweißt, um ein Herauslaufen des flüssigen Wirkstoffes zu vermeiden.

Das bekannte Depotpflaster ist jedoch insofern nachteilig als die Flüssigkeit in diesem Pflaster frei fließt und leicht auslaufen kann, wenn die Klebebzw. Schweißränder beschädigt werden und erfordert zudem eine kostenaufwendige Steuermembran, die zusätzlich zur Wirkstoff-Verteilungseinrichtung vorgesehen sein muß, um die Wirkstoffabgabe kinetisch zu steuern.

Aus der GB-A-1 361 289 ist ein System zur transdermalen Verabreichung von Wirkstoffen an die Haut mit räumlich definiert zueinander angeordneten diskreten Wirkstoffdepots bekannt geworden.

Bei diesen transdermalen System ist ein Reservoir mit einem polymeren Grund- bzw. Stützmaterial, das sich im Pflaster selbst vernetzt oder außerhalb verfestigt werden kann, beschrieben, wobei ein wirkstoffhaltiges Polymer, das in einer Vorstufe erzeugt wurde, verwendet wird.

Aus der EP 117 027 ist ein transdermales System mit einem salbenartigen bzw. hochviskosen Depot bekannt geworden, wobei die Depotkomponenten außerhalb des Pflasters durch Vermischen der Salbengrundlage und des Wirkstoffes hergestellt werden und sodann in das Pflaster eingebracht werden.

Für flüssige bzw. leicht flüchtige Materialien ist die Salbengrundlage gemäß diesem Patent ungünstig, da während der Herstellung stets ein Verdampfen des Wirkstoffmaterials erfolgen wird.

Es ist daher Aufgabe der Erfindung, ein therapeutisches System mit Wirkstoff-Depot für die Verabreichung von Wirkstoff zu schaffen, das preiswerter und sicherer herzustellen ist als die Systeme nach dem Stand der Technik und insbesondere für die Verarbeitung flüchtiger und/oder thermischer instabiler Komponenten geeignet ist.

Die Aufgabe wird erfindungsgemäß durch ein gattungsgemäßes therapeutisches System gelöst, bei dem das Wirkstoffdepot mindestens einen Hilfsstoff mit Stütz- und Verteilungsfunktion in Form eines textilen Flächenmaterials aufweist und allseitig von der Matrix umgeben ist.

Dabei kann die Reservoirmatrix bei der Herstellung des therapeutischen Systems wirkstofffrei sein und sich erst mit der Zeit - während der Lagerung des Systems - oder - bei stark flüchtigen Substanzen - noch während der Fertigstellung des Systems mit dem Wirkstoff anreichern. Es ist also ein Vorteil der Erfindung, daß jetzt auch Wirkstoffe, die thermisch instabil und/oder flüchtig sind, ohne thermische Belastung als Depot in transdermale Systeme während der Herstellung eingebracht werden können.

Schritte, wie Mischen des Reservoirmatrixmaterials mit dem Wirkstoff, entfallen - das Reservoirmatrixmaterial sättigt sich während der Lagerung des therapeutischen Systems mit dem Wirkstoff bei Raumtemperatur. Durch das Wegfallen der Herstellungsschritte für die wirkstoffgesättige Matrix ist die Herstellung stark vereinfacht worden.

Dadurch, daß hier eine Reservoirmatrix mit eigener Steuerfunktion, die u.a. durch die Migrationsgeschwindigkeit des Stoffes durch die Matrix bestimmt wird, eingesetzt wird, kann hier das Vorsehen einer Steuermembran, welches zusätzliche Verfahrensschritte und Membranmaterial bei der Herstellung erfordert, umgangen werden. Dabei kann das Depot aus reinem Wirkstoff bestehen, der fest oder fließfähig sein kann und einen inerten Hilfsstoff, nämlich ein textiles Flächenmaterial und auch noch weitere inerte Hilfsstoffe aufweisen. Unter "inert" soll hier verstanden werden, daß Wirkstoff und Hilfsstoff nicht miteinander reagieren; ein "inerter" Hilfsstoff kann auch ein physiologische Wirkungen besitzender Stoff, wie beispielsweise Dimethylsulfoxid oder dgl. sein, der beispielsweise die Permeabilität der Haut erhöht. Das textile Flächenmaterial macht das Wirkstoff-Depot gegenüber Druck- und Zuganwendung unempfindlich, ferner kann das Depot auch noch weitere Trägerstoffe und dergleichen enthalten.

Als Wirkstoffe können transdermal anwendbare Wirkstoffe eingesetzt werden. Typische Beispiele dafür sind:

- Nicotin
- Corticosteroide: Hydrocortison, Prednisolon, Beclomethason-propionat, Flumethason, Triamcinolon, Triamci nolonacetonid, Fluocinolon, Fluocinolin-acetonid, Fluocinolonacetonidacetat, Clobetasol-propionat, usw.
- Analgetische, anti-inflammatorische Mittel: Acetaminophen, Mefenaminosäure, Flufenaminsäure, Diclofenac, Diclofenac-Natrium-Alclofenac, Oxyphenbutazon, Phenylbutazon, Ibuprofen, Flurbiprofen, Salicylsäure, 1-Menthol, Campher, Sulindac-tolmetin-Natrium, Naproxen, Fenbufen, usw.
- Hypnotisch wirksame Sedativa: Phenobarbital, Amobarbital, Cyclobarbital, Triazolam, Nitrazepam, Lorazepam, Haloperidol, usw.
- Tranquilizer: Fluphenazin, Thioridazin, Lorazepam, Flunitrazepam, Chlorpromazin, usw.
- Antihypertensiva: Pindolol, Indenolol, Nifedipin, Lofexidin, Nipradinol, Bucumolol, usw.
- Antihypertensiv wirkende Diuretica: Hydrothiazid, Bendroflumethiazid, Cyclopenthiazid, usw.
- Antibiotica: Penicillin, Tetracyclin, Oxytetracyclin, Fradiomycinsulfat, Erythromycin, Chloramphenicol, usw.
- Anästhetika: Lidocain, Benzocain, Ethylaminobenzoat, usw.
- Antimikrobielle Mittel: Benzalkoniumchlorid, Nitrofurazon, Nystatin, Acetosulfamin, Clotrimazol, usw.
- Antifungus-Mittel: Pentamycin, Amphotericin B, Pyrrolnitrin, Clotrimazol, usw.
- Vitamine: Vitamin A, Ergocalciferol, Colecalciferol, Octotiamin, Riboflavinbutyrat, usw.
- Antiepileptika: Nitrazepam, Meprobamat, Clonazepam, etc.
- Coronar-Vasodilatatoren: Dipyridamol, Erythrittetranitrat, Pentaerythrittetranitrat, Propatylnitrat, usw.
- Antihistaminika: Diphenylhydramin-hydrochlorid, Chlorpheniramin, Diphenylimidazol, usw.
- Antitussiva: Dextromethorphan (Hydrobromid), Terbutalin (Sulfat), Ephedrin (Hydrochlorid), Salbutanol (Sulfat), Isoproterenol (Sulfat, Hydrochlorid), usw.
- Sexualhormone: Progesteron, usw.
- Thymoleptika: Doxepin, usw.
- Weitere Arzneimittel: 5-Fluoruracil, Fentanyl, Desmopressin, Domperdon, Scopolamin (Hydrobromid), Peptide, usw.

Selbstverständlich ist diese Aufzählung nicht abschließend.

Vorteilhafterweise kann die Wirkstoffreservoir-Matrix schichtweise aufgebaut sein, wobei die Schichten gleich oder unterschiedlich sein können. Die Reservoirmatrix kann haftklebend sein, beispielsweise ein Gummimaterial, wie Styrol/Isopren/Styrol-Blockcopolymerisate, Silicongummi oder auch synthetische Harze, wie Poly-(meth)acrylat, Polyurethan, Polyvinylether, Polyester oder dgl. - eine Zusammenfassung geeigneter Matrixmaterialien findet sich z.B. in der DE-OS 35 00 508, auf die vollinhaltlich Bezug genommen wird. Es kann günstig sein, wenn die Reservoirmatrix haftklebend ist, da dadurch das Vorsehen einer separat haftklebenden Fixierungseinrichtung im System vermieden werden kann; die Verwendung einer derartigen haftklebenden Matrix hängt u.a. von der Verträglichkeit des Matrixmaterials mit dem Wirkstoff ab. Haftklebende Matrixmaterialien sind bekannt.

Bevorzugte nicht-haftklebende Matrixmaterialien sind: Polymerisate bestehend aus Poly(meth)acrylat, Polyvinylpyrrolidon, Ethylcellulose, Hydroxypropylcellulose, Hydroxypropylmethylcellulosephthalat, Polyvinylalkohol bzw. deren Copolymerisate mit Vinyllaurat oder Maleinsäure, Vinylacetat, bzw. dessen Copolymerisat mit Vinyllaurat oder Maleinsäure; Polyvinylether, Butylkautschuk und Polycaprolactam.

Beispielsweise kann dann das Wirkstoff-Depot bzw. mehrere Wirkstoff-Depots zwischen einer rückseitigen Reservoirmatrixschicht und einer hautseitigen Reservoirmatrixschicht eingebracht werden, wobei das Dickenverhältnis der Reservoirmatrixschichten bevorzugt zwischen etwa X:Y = 1:1 bis 1:20 und besonders bevorzugt 1:1 bis 1:5 liegt.

In anderen Fällen kann es sinnvoll sein, wenn die Reservoirmatrix oder aber auch die Reservoirmatrixschichten, aus denen sie aufgebaut ist, mindestens einseitig mit haftklebenden Beschichtungen versehen ist.

Bei einer bevorzugten Ausführungsform der Erfindung kann die Fixierungseinrichtung durch in der Reservoirmatrix eingebettete Klebstoffabschnitte, wie beispielsweise ein umlaufender Kleberand auch Klebepunkte, gebildet sein.

In üblicher Weise ist es möglich, eine ablösbare Schutzschicht für die der Haut zugewandten Flächen des therapeutischen Systems vorzusehen.

Die Summe der Wirkstoffmengen im Depot und Reservoirmatrix liegt vorteilhafter Weise beim bis zum 20-fachen der therapeutisch notwendigen Wirkstoffmenge.

Ein besonders bevorzugtes Verfahren zur Herstellung derartiger Systeme beinhaltet, daß die Reservoirmatrix aus zwei Reservoirmatrixschichten, die gleich oder unterschiedlich sein können, gebildet wird, zwischen die das Wirkstoffdepot eingebracht wird. Dabei können die Reservoirmatrixschichten durch Druck- und/oder Wärmeanwendung zusammengefügt werden. Das Depot kann auch unter Druckanwendung in die Reservoirmatrix eingebracht werden, beispielsweise durch Injektion einer vorherbestimmten Menge oder Eindrücken eines wirkstoffhaltigen Körpers in eine weiche Matrixschicht.

Ein weiteres bevorzugtes Verfahren besteht in der Herstellung mindestens eines Teils des therapeutischen Systems durch Aufstreuen von Partikeln.

Es kann auch eine mehrschichtige Wirkstoffmatrix hergestellt werden. Auch Rück- und Reservoirmatrix-Schicht können durch Druck oder Wärme zusammengefügt werden. Die Reservoirmatrixschicht oder auch -schichten können zumindest teilweise aus flüssigen Materialien hergestellt werden, beispielsweise aus einer Dispersion oder aus der Schmelze oder aus Lösungen.

Das erfindungsgemäßes therapeutische System eignet sich insbesondere für die lokale oder systemische transdermale Wirkstoffverabreichung in der Human- oder Tiermedizin oder läßt sich auch in der Kosmetik einsetzen.

Nachfolgend soll die Erfindung anhand von Ausführungsbeispielen sowie der begleitenden Zeichnung, in der schematisch der Aufbau erfindungsgemäßer therapeutischer Systeme dargestellt ist, erläutert werden. Dabei zeigt:

Fig. 1     einen Schnitt durch eine bevorzugte Ausführungsform eines erfindungsgemäßen therapeutischen Systems;

Fig. 2     einen Schnitt durch eine weitere bevorzugte Ausführungsform des erfindungsgemäßen Systems, bei dem ein Wirkstoffreservoir zwischen Matrixschichten eingebettet ist; und

Fig. 3     einen Schnitt durch ein bahnförmiges Halbfabrikat gemäß der Erfindung.

In Fig. 1 ist ein Schnitt durch ein erfindungsgemäßes therapeutisches System, das auf der Haut 18 durch eine Fixierungseinrichtung 16, beispielsweise eine poröse Haftklebeschicht oder dgl., befestigt ist, schematisch dargestellt. Auf der Fixierungseinrichtung 16 befindet sich die Reservoir-Matrix 12, welche bevorzugt zum Zeitpunkt der Herstellung wirkstofffrei ist (die Wirkstoffsättigung tritt während der Lagerung ein). In die Reservoirmatrix ist ein Depot 14 eingebettet, das hier als ein Wirkstoffkörper 14 dargestellt ist, dessen Wirkstoff sich im Reservoirmatrix-Material löst und durch die Fixierungseinrichtung 16 an die Haut 18 abgegeben wird. Das therapeutische System wird nach außen durch eine Rückschicht 10 abgeschlossen, die für den Wirkstoff und bevorzugt auch für Feuchtigkeit undurchlässig ist und gleichzeitig eine Stützfunktion für das System ausübt.

Fig. 2 zeigt eine weitere bevorzugte Ausführungsform, bei der ein erfindungsgemäßes therapeutisches System, das mittels im Wirkstoffreservoir-Matrixmaterial hautseitig eingebetteter Klebstoffpartikel bzw. -abschnitte an der Haut 18 befestigt ist. Die Wirkstoff-Reservoirschicht 12 ist hier aus einer oberen Schicht X und einer unteren Schicht Y hergestellt, zwischen die der Wirkstoff, hier beispielsweise in flüssiger Form, eingebracht ist. Das Vorsehen zweier Reservoirmatrix-Schichten X, Y ist dann vorteilhaft, wenn die Herstellung eines derartigen Systems erfolgt, daß zunächst die untere Wirkstoffreservoir-Schicht vorgelegt wird - ggf. bereits mit aufkaschierter Deckfolie o.ä. - danach nach einem vorherbestimmten Muster das Wirkstoff-Material aufgebracht, die nächste Wirkstoffreservoir-Schicht X darüber gelegt und in üblicher Weise durch Aufbringen der Rückschicht bzw. ggf. diverser Klebeschichten das System vervollständigt wird. Es kann aber auch sinnvoll sein, zunächst beide Wirkstoff-Reservoirschichten X, Y aufeinander zu legen, sodann eine vorherbestimmte Menge Wirkstoff zwischen die beiden Reservoirschichten einzuspritzen und derart die Verdunstung des Wirkstoffes auf einem Minimum zu halten.

In Fig. 3 ist nun der Vorläufer eines erfindungsgemäßen transdermalen Systems dargestellt, wie er während eines bevorzugten Herstellungsverfahrens anfällt. Ein bahnförmiges Schutzschichtmaterial, wie beispielsweise gewachstes Papier od. dgl. ist von einer Reservoirmatrix-Schicht Y, die hier haftklebend ausgebildet ist, überlagert, auf der sich Wirkstoffdepotkörper entsprechend einem vorherbestimmten Muster angeordnet, befinden. Die Matrixschicht Y wird von einer zweiten Matrixschicht X, die beispielsweise auch aus einem anderen Material als die Schicht Y bestehen kann, überlagert. Die zweite Matrixschicht Y wird durch eine Rückschichtfolie 10 abgeschlossen. Entlang der Pfeile befinden sich die Trennlinien, entlang derer das Zwischenprodukt bei der Herstellung der erfindungsgemäßen transdermalen Systeme geschnitten/gestanzt und anschliessend in üblicher Weise konfektioniert wird.

Bei speziellen Anwendungsformen ist es auch möglich, die als "Halbfabrikat" bezeichnete Anordnung als solche in den Handel zu bringen, um es Benutzern zu ermöglichen, das Abtrennen der Systeme selbst vorzunehmen, so daß das Halbfabrikat als eine Art "Vorratspackung" wirkt.

Typische Dickenabmessungen für erfindungsgemäße transdermale Systeme sind: bei einer Gesamtdicke von ca. 123 um bis 5550 $\mu$m, vorzugsweise 285 $\mu$m - 1550 $\mu$m; Dicke der Rückschicht: 8 - 150 $\mu$m, vorzugsweise 15 - 100 $\mu$m; Dicke des Reservoirs: 100 - 5000 $\mu$m, vorzugsweise 200 - 1300 $\mu$m; Dicke der Schutzschicht: 15 - 400 $\mu$m, vorzugsweise 70 - 150 $\mu$m.

Nachfolgend sollen nun bevorzugte Ausführungsbeispiele der Erfindung erläutert werden.

Beispiel 1

Herstellung eines Nikotinpflasters:

Nikotinpflaster, wie sie beispielsweise für die Raucherentwöhnung einsetzbar sind, können erfindungsgemäß, wie folgt, hergestellt werden:

Eine Haftklebemasse, bestehend aus 2,0825 kg einer 40%igen Lösung eines selbstvernetzenden Acrylatpolymeren (selbstvernetzendes Acrylatcopolymeres aus 2-Ethylhexylacrylat, Vinylacetat, Acrylsäure und Titanchelatester, unter der Bezeichnung Durotak 280 - 2416 der Fa. National Starch & Chemical B.V im Handel erhältlich) in einer Mischung aus Essigsäureethylester, Ethanol, Hexan und Methanol, 147 g eines Acrylharzes aus Dimethylaminoethylmethacrylat und neutralem Methacrylsäureester (Eudragit E 100 der Fa. Röhm-Pharma) sowie 20 g eines gemischt sauren Triglycerids der fraktionierten Kokosfettsäuren $C_8$-$C_{10}$ (Miglyol 812 der Fa. Dynamit Nobel) werden auf eine einseitig mit Aluminium bedampfte und beidseitig abhäsiv ausgerüstete Schutzschicht aufgetragen und das Lösungsmittel bei 50 - 80°C verdampft. Es wird eine Schicht von ungefähr 300 g/m² erhalten. Aus der derart hergestellten Hattklebeschicht werden Ronden mit einem Durchmesser von 65 mm gestanzt, die überstehenden Ränder abgegittert und mittig auf dieser jeweils eine Ronde aus einem Vliesstoff (Fasergemisch Zellwolle/Baumwolle 50 : 50 mit einem Flächengewicht von 80 g/m², Paratex II/80 der Fa. Lohmann GmbH & Co. KG) mit einem Durchmesser von 40 mm aufkaschiert. Darauf wird Nikotin als Wirkstoff in Lösung (140 g Nikotin in 100 g eines Acrylharzes aus Dimethylaminoethylmethacrylat und neutralen Methacrylsäureestern, Eudragit E 100 der Fa. Röhm Pharma) in 102 mg-Dosen/Ronde aufgebracht. Die derart hergestellten "Patches" werden sofort mit einer nikotinundurchlässigen Rückschicht (einseitig Aluminiumbedampfte Polyesterfolie, mit 15 $\mu$m Dicke, laminiert und in Vierrandsiegelbeutel aus bekanntem Verbundpackstoff eingesiegelt.

Bei diesem Ausführungsbeispiel wirkt der Vliesstoff als Stützgewebe bzw. zur Unterstützung der gleichmäßigen Verteilung des Nikotins als inerter Hilfsstoff im Sinne der Beschreibungseinleitung.

Dadurch, daß erfindungsgemäß eine Wirkstofflösung schnell auf eine Matrixschicht aufgebracht werden kann und so gleich von einer Wirkstoffundurchlässigen Deckschicht überzogen wird, ist es erstmals möglich, in zufriedenstellender Weise gut dosierte Nikotinpflaster zu erhalten.

Nikotin-Freisetzungsversuch:(in vitro)

Ein, wie in Beispiel 1 hergestelltes Nikotinpflaster wird nach Abziehen der Schutzschicht in 80 ml isotonische Kochsalzlösung bei 37°C eingetaucht und die freigesetzte Nikotinmenge nach festgelegten Zeitabständen flüssigkeitschromatographisch bestimmt. Das Volumen des Freigabemediums wurde so gewählt, daß über die gesamte Versuchsdauer "sink" Bedingungen eingehalten werden.

Es wurden folgende Messungen erzielt:
In vitro-freigesetztes Nikotin/Pflaster:

nach 2 Stunden:

23,90 mg/Pflaster

nach 4 Stunden:

32,34 mg/Pflaster

nach 8 Stunden:

41,50 mg/Pflaster

nach 24 Stunden:

56,54 mg/Pflaster

Beispiel 2

Herstellung eines Nikotinpflasters:

Ein weiteres erfindungsgemäßes Nikotinpflaster kann, wie folgt, hergestellt werden:
Eine Haftklebemasse (Haftklebemasse 1), bestehend aus 1,9758 kg einer 40 %igen Lösung eines selbstvernetzenden Acrylatpolymerem (Durotak 280 -2516 der Firma Delft National & Chemical B.V.) in einer Mischung aus Essigsäureethylester, Ethanol, Heptan und Methanol) 189,7 g eines Acrylharzes aus Dimethylaminoethylmethacrylat und neutralem Methacrylsäureester (Eudragit E 100 der Fa. Röhm Pharma) sowie 20 g eines gemischt sauren Triglycerids der fraktionierten Kokosfettsäuren $C_8$ - $C_{10}$ - (Miglyol 812 der Fa. Dynamit Nobel) werden auf eine einseitig mit Aluminium bedampfte und beidseitig abhäsiv ausgerüstete Schutzschicht aufgetragen und das Lösungsmittel bei 50 - 80° Celsius verdampft. So wird eine Schicht von ungefähr 440 g/m$^2$ erhalten. Aus der derart hergestellten Haftklebeschicht werden Ronden mit einem Durchmesser von 51 mm gestanzt, die überstehenden Ränder abgegittert und mittig auf dieser jeweils eine Ronde aus einem Vlies-Stoff (Fasergemisch Zellwolle/Baumwolle 70 : 30 mit einem Flächengewicht von 40 g/m$^2$, Paratex III/40 der Fa. Lohmann GmbH & Co. KG) mit einem Durchmesser von 42 mm aufkaschiert.

Darauf wird Nicotin als Wirkstoff in Lösung (140 g Nikotin in 100g eines Acrylharzes aus Dimethylaminoethylmethacrylat und neutralen Methacrylsäureestern, Eudragit E 100 der Fa. Röhm) in 46 mg-Dosen/Ronde aufgebracht. Die derart hergestellten "Patches" werden sofort laminiert mit einer nicotinundurchlässigen Rückschicht (einseitig Aluminiumbedampfte Polyesterfolie mit 15 μm Dicke, die beschichtet ist mit ca. 110 g/m$^2$ Haftklebemasse 1) und in Vierrandsiegelbeutel aus an sich bekanntem, geeignetem Verbundpackstoff eingesiegelt.

Bei diesem Ausführungsbeispiel wirkt der Vliesstoff als Stützgewebe bzw. zur Unterstützung der gleichmäßigen Verteilung des Nikotins als inerter Hilfsstoff im Sinne der Beschreibungseinleitung.

Dadurch, daß erfindungsgemäß eine Wirkstofflösung schnell auf eine Matrixschicht aufgebracht werden kann und sogleich von einer wirkstoffundurchlässigen Deckschicht überzogen wird, ist es erstmals möglich, in zufriedenstellender Weise gut dosierte Nikotinpflaster zu erhalten.

Nikotin-Freisetzungsversuch:(in vitro)

Ein wie in Beispiel 2 beschrieben, hergestelltes Nikotinpflaster wird nach Abziehen der Schutzschicht in 80 ml isotonische Kochsalzlösung bei 37°C eingetaucht und die freigesetzte Nikotinmenge nach festgelegten Zeitabständen flüssigkeitschromatographisch bestimmt. Das Volumen des Freigabemediums wurde so gewählt, daß über die gesamte Versuchsdauer "sink" Bedingungen eingehalten werden.

Es wurden folgende Messungen erzielt:
In vitro-freigesetztes Nikotin/Pflaster:

nach 2 Stunden:

5,1 mg/Pflaster

nach 4 Stunden:

7,2 mg/Pflaster

nach 8 Stunden:

10,1 mg/Pflaster

nach 24 Stunden:

16,5 mg Pflaster

Selbstverständlich ist die Erfindung nicht nur auf die Herstellung von Nicotinpflastern bzw. auf Nicotinpflaster mit erfindungsgemäßem Pflasteraufbau beschränkt; es können, wie weiter oben beschrieben, auch beliebige andere Wirkstoffe, von denen eine bevorzugte Auswahl in der Beschreibung aufgeführt wurde, durch dieses neue therapeutische System abgegeben werden.

**Patentansprüche**

1. Therapeutisches System zur Verabreichung von Wirkstoffen an die Haut mit einer der Haut abgewandten Rückschicht; einem flüssigen Wirkstoff oder flüssige Wirkstoffzubereitung enthaltenden Wirkstoffdepot; einer die Wirkstoffabgabe steuernden Matrix und einer haftklebenden Fixierungseinrichtung für das therapeutische System auf der Haut, dadurch gekennzeichnet,daß das Wirkstoffdepot (14) mindestens einen Hilfsstoff mit Stütz- und Verteilungsfunktion in Form eines textilen Flächenmaterials aufweist und allseitig von der Matrix (12) umgeben ist.

2. Therapeutisches System nach Anspruch 1, dadurch gekennzeichnet, daß das textile Flächenmaterial ein Gewebe oder ein Vliesstoff ist.

3. Therapeutisches System nach Anspruch 1, dadurch gekennzeichnet, daß das therapeutische System mehrere Wirkstoffe aufweist.

4. Therapeutisches System nach einem der vorangehenden Ansprüche, dadurch gekennzeichnet, daß die Matrix (12) aus mindestens zwei Schichten besteht, wobei bevorzugt zwischen einer rückseitigen Matrixschicht (X) und einer hautseitigen Matrixschicht (Y) ein oder mehrere Wirkstoffdepot(s) (14) eingebracht ist/sind, wobei das Dickenverhältnis der Matrixschichten bevorzugt zwischen etwa X:Y = 1:1 bis 1:20 und besonders bevorzugt zwischen 1:1 bis 1:5 liegt.

5. Therapeutisches System nach einem der vorangehenden Ansprüche, dadurch gekennzeichnet, daß die Matrix (12) haftklebend ist.

6. Therapeutisches System nach einem der vorangehenden Ansprüche, dadurch gekennzeichnet, daß die Matrix (12) oder eine oder mehrere Matrixschichten (X,Y) mindestens einseitig haftklebende Einrichtungen (16) aufweisen.

7. Therapeutisches System nach einem der vorangehenden Ansprüche, dadurch gekennzeichnet, daß die Fixierungseinrichtung (16) in die Matrix (12) eingebettete Klebstoffabschnitte ist.

8. Therapeutisches System nach einem der vorangehenden Ansprüche, dadurch gekennzeichnet, daß es eine ablösbare Schutzschicht (19) für die der Haut zugewandten Flächen aufweist.

9. Therapeutisches System nach einem der vorangehenden Ansprüche, dadurch gekennzeichnet, daß der Wirkstoff Nicotin ist.

10. Verfahren zur Herstellung eines therapeutischen Systems nach einem der vorangehenden Ansprüche, dadurch gekennzeichnet, daß das Wirkstoffdepot bei der Herstellung des therapeutischen Systems in situ durch Vereinigung von Depotkomponenten hergestellt wird.

11. Verfahren zur Herstellung eines therapeutischen Systems nach einem der vorangehenden Ansprüche, dadurch gekennzeichnet, daß die Matrix-Schichten durch Druck-und/oder Wärmeanwendung zusammengefügt werden.

12. Verfahren zur Herstellung eines therapeutischen System nach einem der vorangehenden Ansprüche, dadurch gekennzeichnet, daß Rückschicht und Matrix durch Druck und/oder Wärme zusammengefügt werden.

13. Verfahren zur Herstellung eines therapeutischen Systems nach einem der vorangehenden Ansprüche, dadurch gekennzeichnet, daß die Matrix aus mindestens zwei Matrix-Schichten, die gleich oder unterschiedlich sein können, gebildet wird, zwischen die das Wirkstoffdepot eingebracht wird.

14. Verfahren zur Herstellung eines therapeutischen Systems nach einem der vorangehenden Ansprüche, dadurch gekennzeichnet, daß das Depot durch Druckanwendung in die Matrix eingebracht wird.

15. Verfahren zur Herstellung eines therapeutischen Systems nach einem der vorangehenden Ansprüche, dadurch gekennzeichnet, daß mindestens ein Teil des therapeutischen Systems aus Lösungen oder aus einer Dispersion oder aus der Schmelze oder durch Aufstreuen von Partikeln hergestellt wird.

16. Verwendung des therapeutischen Systems nach einem der Ansprüche 1 bis 9 für die lokale oder systemische transdermale Wirkstoffverabreichung in der Kosmetik.

**Claims**

1. Therapeutic system for applying active substances to the skin, comprising a backing layer directed away from the skin, an active substance depot containing a liquid active substance or a liquid formulation of active substance, a matrix controlling the active substance release, and a pressure sensitive adhesive arrangement for fixing the therapeutic system to the skin, characterised in that the active substance depot (14) has at least one auxiliary substance with a supporting and distributing function in the form of a flat textile material and is surrounded on all sides by the matrix (12).

2. Therapeutic system according to Claim 1, characterised in that the flat textile material is a woven or a nonwoven fabric.

3. Therapeutic system according to Claim 1, characterised in that the therapeutic system has a number of active substances.

4. Therapeutic system according to one of the preceding claims, characterised in that the matrix (12) consists of at least two layers, one or more active substance depots (14) being introduced preferably between a rear-side matrix layer (X) and a skin-side matrix layer (Y), the thickness ratio of the matrix layers lying preferably between about X:Y = 1:1 to 1:20 and particularly preferably between 1:1 to 1:5.

5. Therapeutic system according to one of the preceding claims, characterised in that the matrix (12) is pressure sensitive adhesive.

6. Therapeutic system according to one of the preceding claims, characterised in that the matrix (12) or one or more matrix layers (X,Y) have arrangements (16) which are pressure sensitive adhesive at least on one side.

7. Therapeutic system according to one of the preceding claims, characterised in that the fixing arrangement (16) is pressure-sensitive adhesive sections embedded in the matrix (12).

8. Therapeutic system according to one of the preceding claims, characterised in that it has a detachable protective layer (19) for the surfaces directed towards the skin.

9. Therapeutic system according to one of the preceding claims, characterised in that the active substance is nicotine.

10. Method for producing a therapeutic system according to one of the preceding claims, characterised in that the active substance depot is prepared in situ, during production of the therapeutic system, by combination of depot components.

11. Method for producing a therapeutic system according to one of the preceding claims, characterised in that the matrix layers are joined together by application of pressure and/or heat.

12. Method for producing a therapeutic system according to one of the preceding claims, characterised in that backing layer and matrix are joined together by pressure and/or heat.

13. Method for producing a therapeutic system according to one of the preceding claims, characterised in that the matrix is made up of at least two matrix layers, which can be identical or different and between which the active substance depot is introduced.

14. Method for producing a therapeutic system according to one of the preceding claims, characterised in that the depot is introduced into the matrix by pressure application.

15. Method for producing a therapeutic system according to one of the preceding claims, characterised in that at least a part of the therapeutic system is produced from solutions or from a dispersion or from the melt or by scattering of particles.

16. Use of the therapeutic system according to one of Claims 1 to 9 for the local or systemic transdermal application of active substances in cosmetics.

**Revendications**

1. Système thérapeutique permettant d'administrer des principes actifs par la peau, comprenant une couche postérieure opposée à la peau, un dépôt contenant un principe actif liquide ou une préparation de principe actif liquide, une matrice commandant la libération du principe actif et un dispositif de fixation autocollant enlevable permettant de fixer le système thérapeutique sur la peau, caractérisé en ce que le dépôt de principe actif (14) comporte au moins une matière auxiliaire qui a une fonction d'appui et de distribution sous la forme d'un matériau textile plat et est entourée de toutes part par la matrice (12).

2. Système thérapeutique selon la revendication 1, caractérisé en ce que le matériau textile plat est un tissu ou une matière non tissée.

3. Système thérapeutique selon la revendication 1, caractérisé en ce qu'il comporte plusieurs principes actifs.

4. Système thérapeutique selon l'une quelconque des revendications précédentes, caractérisé en ce que la matrice (12) est constituée d'au moins deux couches, un ou plusieurs dépôts de principe actif (14) étant agencé(s) de préférence entre la couche postérieure (X) de la matrice et la couche côté peau (Y) de la matrice et le rapport des épaisseurs des couches de la matrice X:Y étant de préférence compris entre environ 1:1 et 1:20, mieux encore entre 1:1 et 1:5.

5. Système thérapeutique selon l'une quelconque des revendications précédentes, caractérisé en ce que la matrice (12) est constituée d'un autocollant enlevable.

6. Système thérapeutique selon l'une quelconque des revendications précédentes, caractérisé en ce que la matrice (12) ou une plusieurs des couches (X,Y) de la matrice comportent des dispositifs autocollants enlevables (16) au moins sur un côté.

7. Système selon l'une quelconque des revendications précédentes, caractérisé en ce que les dispositifs de fixation (16) sont constitués de sections de matière collante noyées dans la matrice (12).

8. Système thérapeutique selon l'une quelconque des revendications précédentes, caractérisé en ce qu'il présente une couche de protection séparable (19) pour les surfaces tournées vers la peau.

9. Système thérapeutique selon l'une quelconque des revendications précédentes, caractérisé en ce que le principe actif est la nicotine.

10. Procédé de fabrication d'un système thérapeutique selon l'une quelconque des revendications précédentes, caractérisé en ce que le dépôt de principe actif est réalisé par combinaison de ses composants in situ lors de la fabrication du système thérapeutique.

11. Procédé de fabrication d'un système thérapeutique selon l'une quelconque des revendications précédentes, caractérisé en ce que les couches de la matrice sont assemblées par application de pression et/ou de chaleur.

12. Procédé de fabrication d'un système thérapeutique selon l'une quelconque des revendications précédentes, caractérisé en ce que la couche postérieure et la matrice sont assemblées par application de pression et/ou de chaleur.

13. Procédé de fabrication d'un système thérapeutique selon l'une quelconque des revendications précédentes, caractérisé en ce que la matrice est formée d'au moins deux couches qui peuvent être identiques ou différentes et entre lesquelles est agencé le dépôt de principe actif.

14. Procédé de fabrication d'un système thérapeutique selon l'une quelconque des revendications précédentes, caractérisé en ce que le dépôt est agencé dans la matrice par application de pression.

15. Procédé selon l'une quelconque des revendications précédentes, caractérisé en ce qu'au moins une partie du système thérapeutique est constituée de solutions ou d'une dispersion ou encore d'une matière fusionnée, voire de particules disséminées.

16. Usage du système thérapeutique selon l'une quelconque des revendications 1 à 9 pour l'administration transcutanée locale ou systémique de principes actifs en cosmétique.